# EUROPÄISCHE PATENTANMELDUNG

(11) **EP 1 908 453 A1**
(43) Veröffentlichungstag der Anmeldung: **09.04.2008**
(21) Anmeldenummer: 07017619.3
(22) Anmeldetag: 08.09.2007
(51) Int. Cl.: A61K 8/37, A61Q 19/10, A61P 17/10, A61K 31/22

(54) **Alkylethercitrate für die selektive Reinigung der Haut**

(30) Priorität: 19.09.2006 DE 102006044618
(71) Anmelder: Cognis IP Management GmbH, 40589 Düsseldorf (DE)
(72) Erfinder: Mehling, Annette, 42349 Wuppertal (DE); Kopp-Holtwiesche, Bettina, 40599 Düsseldorf (DE); Hensen, Hermann, 42781 Haan (DE)

(57) **Zusammenfassung**

Vorgeschlagen wird die Verwendung von Alkylethercitraten in kosmetischen und pharmazeutischen Zubereitungen zur Aufrechterhaltung und zum Schutz und zur Aufrechterhaltung der natürlichen Haut-/Schleimhautmikroflora und des mikrobiellen Haut-/Schleimhaut-Ökosystems, wobei die natürlichen Balanceprozesse, die die Menge an pathogenen Bakterien derart einschränken, dass diese nicht krankheitsauslösend werden, nicht beeinträchtigt werden.

## Beschreibung

### Gebiet der Erfindung

Die Erfindung befindet sich auf dem Gebiet der kosmetischen und pharmazeutischen Hautreinigungsmittel und insbesondere der selektiven Reinigung der Hautoberfläche mit Hilfe von bestimmten Alkylethercitraten.

### Stand der Technik

Reinigung der Haut zielt seit jeher darauf ab, die Haut von anhaftender Verschmutzung zu befreien und so u.a. die Übertragung von infektiösen Bakterien und Pilzen zu vermeiden. Zu häufiger Gebrauch von Tensiden oder antiseptischen Zubereitungen, wie z.B. Duschbädern, Duschgelen, Spülmitteln, Shampoos etc., führt zur Schädigung der Haut. Die hauteigenen Lipide werden dabei genauso wie die Verschmutzungen entfernt und führen zu einem Austrocknen der Haut. Die Haut wird rau und trocken. Ausserdem wird die natürliche Hautflora durch häufiges Waschen zerstört oder in ihrer Zusammensetzung geändert, d.h. die Keime, die für ein gesundes Hautmilieu verantwortlich sind, werden zerstört und/oder entfernt. Die Hautmikroflora spielt auch eine wesentliche Rolle in der Aufrechterhaltung des Säureschutzmantels der Haut und Schleimhaut. Sobald der natürliche Säureschutzmantel der Haut angegriffen oder sogar zerstört ist, besteht die Gefahr, dass pathogene Keime die Haut besiedeln bzw. die Oberhand über die "natürliche" Hautflora erlangen und zu Entzündungen führen. Durch veränderte Homeostase können Vertreter von residenten Mikroorganismen pathogene Eigenschaften entwickeln. Die natürliche Hautflora besteht aus einem heterogenen Spektrum von Bakterien und Hefen/Pilzen, wobei vorrangig grain-positive Bakterien (wie z.B. Staphylococcus epidermidis, Propionibacterium acnes) oder die Hefen der Malassezia-Gruppe vorhanden sind. Die Zusammensetzung der Hautmirkoflora jeder einzelnen Person ist zudem abhängig von Körperlokalisation und Lebensbedingungen.

Aufgabe der vorliegenden Erfindung war es, Tensidformulierungen zu finden, die die natürliche Hautmikroflora nicht beeinflussen oder nur in geringem Maße, da eine gesunde Hautmikroflora eine bedeutende Rolle bei der Hautbarrierefunktion spielt. Gleichzeitig sollen diese Formulierungen jedoch in der Lage sein, die Haut effektiv von anhaftenden Verschmutzungen zu befreien.

### Beschreibung der Erfindung

Gegenstand der vorliegenden Erfindung ist die Verwendung von Alkylethercitraten als selektive Tenside zur Hautreinigung. Unter selektiven Tensiden werden in diesem Zusammenhang Tenside verstanden, die die natürlich auf der Haut vorkommenden Keime (Bakterien und Hefen/Pilze) nicht oder nur in geringem Maße angreifen. Diese Tenside werden zum Schutz und zur Aufrechterhaltung der natürlichen Haut-/Schleimhautmikroflora und des mikrobiellen Haut-/Schleimhaut-Ökosystems eingesetzt, wobei die natürlichen Balanceprozesse, die die Menge an pathogenen Bakterien derart einschränken, dass diese nicht krankheitsauslösend werden, nicht beeinträchtigt werden. Sie dienen dem Schutz der Haut und der Hautbarrierefunktion sowie der Aufrechterhaltung der Homeostase. Sie werden verwendet zur Aufrechterhaltung des natürlichen pH-Wertes (Säureschutzmantel der Haut) und zur Kontrolle der Besiedelung der Haut mit pathogenen Keimen und dienen somit der Aufrechterhaltung des natürlichen Hautökosystems.

In diesem Zusammenhang bezieht sich der Begriff Haut sowohl auf Haut als auch auf Schleimhaut. Unter Hautmikroflora/Hautflora/Standflora werden die die Haut besiedelnden Mikroorganismen (Bakterien, Hefen, Pilze, Viren), das mikrobielle Ökosystem verstanden. Sie stellen das mikrobielle Ökosystem der Haut dar, dem eine Barrierefunktion zukommt. Unter den Begriff Hautreinigung werden zum Beispiel folgende kosmetische und pharmazeutische Mittel zusammengefasst: Körperreinigungsmittel, Intimpflege, Duschbäder, Duschöle, Duschgele, Shampoos, Conditioner, Schaumbäder, Babyshampoos, trockene und feuchte Reinigungstücher, Cremes und Lotions etc.

### Alkylethercitrate

Unter Alkylethercitraten werden Citronensäureestermischungen von ethoxylierten Alkoholen der allgemeinen Formel (I)

R¹O(CH₂CH₂O)nH (I)

verstanden, in der R¹ für einen Alkylrest und n für den Ethoxylierungsgrad steht, wobei R¹ für einen linearen Alkylrest mit 6 bis 22 Kohlenstoffatomen steht. Dieser Alkylrest kann sich jedoch auch von einer Fettlalkoholmischung natürlichen oder synthetischen Ursprungs mit verschiedenen Alkylkettenlängen ableiten. In einer bevorzugten Ausführungsform ist der Alkylrest R¹ abgeleitet von einer Fettalkoholmischung enthaltend 45 - 75 Gew.% C12-, 15 bis 35 Gew.% C14-, 0 - 15 Gew.% C16- und 0 bis 20 Gew.% C18- Alkohol. n steht für Zahlen von 5 bis 9. Das Gewichtsverhältnis von Monoester : Diester liegt bevorzugt in den Citronensäureestermischungen im Bereich von 3: 1 bis 10 : 1.

### Beispiele

Das Ausmaß einer antimikrobiellen Wirkung von Tensiden läßt sich in einem biologischen Testsystem feststellen, wobei die "minimale Hemmkonzentration bzw. Inhibierungskonzentration" (MIC) unter Laborbedingungen vergleichend bestimmt wird. In einer Verdünnungsreihe der Testsubstanz ist diejenige Konzentration die MIC, bei der gerade kein Wachstum mehr möglich ist. Je milder ein Tensid ist, umso größer ist der MIC-Wert.

Die mittlere Inhibierungskonzentration verschiedener Tenside wurde für drei auf der Haut vorkommenden Mikroorganismen bestimmt (Tabelle 1). Die Tenside wurden in der INCI-Nomenklatur angegeben.

**Tabelle 1: Minimale Inhibierungskonzentration verschiedener Tenside Testlösung: 10% Aktivsubstanz, pH 6,5 Bestimmunggemäß DIN 58940 und DIN 58944.**

| | **Sodium Laureth Sulfate** | **Cocamidopropylbetaine** | **Lauryl Glucoside** | **Laureth-7 Citrate** |
|---|---|---|---|---|
| Propionibacterium acnes | 0,05 | 0,05 | 0,05 | >0,5 |
| Staphylococcus epidermidis | 0,5 | 0,1 | 0,5 | >0,5 |
| Malassezia furfur | >0,5 | >0,5 | >0,5 | >0,5 |

Das Alkylethercitrat Laureth-7 citrate zeichnet sich gegenüber den anderen Tensiden dadurch aus, dass Propionibacterium acnes und Staphylococcus epidermidis erst bei wesentlich höheren Konzentrationen inhibiert werden.

Bei Verwendung von Alkylethercitraten in Kombination mit anderen Tensiden hat die Selektivität der Alkylethercitrate Einfluß auf das Keimtötungspotential der Gesamtformulierung, d.h. die positiven Eigenschaften der Alkylethercitrate auf die Aufrechterhaltung der Homeostase bzw. die Wachstumseigenschaften der Mikroflora wirken sich auch auf die Gesamtzusammensetzung aus.
Statt die Hautabwehr gegenüber Pathogenen zu mindern, wie es herkömmliche Tenside oft tun, bleibt sie unverändert.

Tabelle 2 führt minimalen Inhibierungskonzentrationen verschiedener Mischungen von Alkylethercitraten mit Alkylethersulfaten auf. Dabei wird deutlich, dass bei gleichem Aktivsubstanzgehalt die Formulierungen mit steigendem Gehalt an Alkylethercitraten steigende minimale Inhibierungskonzentrationen aufweisen, d.h. über ein vermindertes Irritationspotential verfügung und somit zur Aufrechterhaltung oder Wiederherstellung einer natürlichen und gesunden Hautflora beitragen.

**Tabelle 2: Minimale Inhibierungskonzentration verschiedener Tensidekombinationen Testlösung: 10% Aktivsubstanz Bestimmunggemäß DIN 58940 und DIN 58944. AEC = Alkylethercitrat = Laureth-7 citrate AES = Alkylethersulfat = Sodium Laureth Sulfate**

| | **AEC 100%** | **AEC/AES 75/25 %** | **AEC/AES 50/50 %** | **AEC/AES 25/75 %** | **AES 100 %** |
|---|---|---|---|---|---|
| Propionibacterium acnes | ≥ 1 | ≥ 1 | 0,5 | 0,1 | 0,05 |
| Staphylococcus epidermidis | ≥ 1 | ≥1 | ≥1 | 1,0 | 0,5 |
| Malassezia furfur | ≥ 1 | ≥ 1 | ≥ 1 | ≥1 | ≥ 1 |

## Patentansprüche

1. Verwendung von Alkylethercitraten als selektive Tenside zur Hautreinigung.

2. Verwendung von Alkylethercitraten zum Schutz und zur Aufrechterhaltung der natürlichen Haut-/Schleimhautmikroflora und des mikrobiellen Haut-/Schleimhaut-Ökosystems, wobei die natürlichen Balanceprozesse, die die Menge an pathogenen Bakterien derart einschränken, dass diese nicht krankheitsauslösend werden, nicht beeinträchtigt werden.

3. Verwendung von Alkylethercitraten zum Schutz der Haut und der Hautbarrierefunktion und Aufrechterhaltung der Homeostase.

4. Verwendung von Alkylethercitraten zur Aufrechterhaltung des natürlichen pH-Wertes des Säureschutzmantels der Haut und Kontrolle der Besiedelung mit pathogenen Keimen.

5. Verwendung von Alkylethercitraten zur Aufrechterhaltung des natürlichen Hautökosystems.

6. Verwendung gemäß wenigstens einem der Ansprüche 1 bis 5, **dadurch gekennzeichnet, dass** als Alkylethercitrate Citronensäureestermischungen von ethoxylierten Alkoholen der allgemeinen Formel (I)
R¹O(CH₂CH₂O)nH (I)
verwendet werden, in der R¹ für einen Alkylrest mit 6 bis 22 Kohlenstoffatomen und n für den Ethoxylierungsgrad mit Zahlen von 5 bis 9 steht.

7. Verwendung gemäß einem der Ansprüche 1 bis 6, **dadurch gekennzeichnet, dass** sich der Alkylrest R¹ ableitet von einer Fettalkoholmischung enthaltend 45 - 75 Gew.% C12-, 15 bis 35 Gew.% C14-, 0 - 15 Gew.% C16- und 0 bis 20 Gew.% C18- Alkohol.

8. Verwendung gemäß einem der Ansprüche 1 bis 7, **dadurch gekennzeichnet, dass** Gewichtsverhältnis von Monoester : Diester in den Citronensäureestermischungen im Bereich von 3: 1 bis 10 : 1 liegt.
